# FASCICULE DE BREVET EUROPEEN

(11) **EP 0 993 281 B1**
(45) Date de publication et mention de la délivrance du brevet: **11.02.2004**
(21) Numéro de dépôt: 99916968.3
(22) Date de dépôt: 30.04.1999
(51) Int. Cl.: A61F 2/16

(54) **IMPLANT INTRAOCULAIRE**
INTRAOKULÄRES IMPLANTAT
INTRAOCULAR IMPLANT

(30) Priorité: 04.05.1998 FR 9805605
(43) Date de publication de la demande: 19.04.2000
(73) Titulaire: HumanOptics AG, 90542 Eckental (DE)
(72) Inventeur: Hanna, Khalil, 75007 Paris (FR)
(74) Mandataire: Robert, Jean-Pierre
(86) Numéro de dépôt international: PCT/FR1999/001037
(87) Numéro de publication internationale: WO 1999/056670

(56) Documents cités:
- EP-A- 0 337 390
- EP-A- 0 507 292
- EP-A- 0 732 090
- EP-A- 0 779 063
- US-A- 4 878 910
- US-A- 5 628 798

## Description

La présente invention concerne un implant intraoculaire destiné principalement à remplacer le cristallin naturel en ses lieu et place, après opération de la cataracte, c'est-à-dire à l'intérieur du sac capsulaire formant l'enveloppe du cristallin naturel.

La technique opératoire de la cataracte la plus utilisée à ce jour est celle dite de phaco-émulsification, à travers un orifice circulaire de 5 à 6 mm de diamètre ménagé dans la face antérieure de la capsule (capsulorhexis). Elle permet de retirer le contenu cristallinien préservant l'intégrité du reste de l'enveloppe (sac) capsulaire pour pouvoir y placer un implant cristallinien. La phaco-émulsification a comme avantage de n'avoir besoin que d'une incision minimale pour l'introduction dans l'oeil de la sonde nécessaire à l'ablation de la matière cristalline. On comprend l'intérêt qu'il y a alors de profiter de cette petite incision pour mettre en place l'implant.

Or pour qu'un implant puisse être maintenu dans le sac capsulaire, il faut qu'il possède une dimension suffisante afin d'assurer un bon centrage de l'optique, même après une rétraction capsulaire. Pour cette raison le diamètre total de l'implant est de l'ordre de 11 à 12 mm. Par ailleurs, il est pratiquement impossible et en tout cas mal commode, même avec des implants souples et pliables, de conserver cette petite incision que le chirurgien agrandit le plus souvent, au détriment des propriétés optiques de la cornée dans laquelle elle est réalisée.

En outre, dans les implants connus, la partie haptique de l'implant n'appuie pas de manière uniforme sur la partie équatoriale du sac capsulaire, ce qui conduit à l'apparition de plis de sa partie postérieure, qui provoquent une baisse de l'acuité visuelle même sans fibrose excessive. Il faut alors le plus souvent avoir recours à une intervention ultérieure par laquelle on procède à un découpage de la capsule postérieure par laser YAG.

Parfois, la puissance de l'implant ne correspond pas aux besoins du malade. Or du fait des adhérences de la partie haptique au sac capsulaire qui en général s'est refermé sur elles, il y a un risque à procéder au changement chirurgical de l'implant. Pour tenter de corriger le système optique du patient, on a recours quelquefois à la chirurgie réfractive cornéenne.

Dans certains cas, le bord de l'ouverture antérieure du sac capsulaire (ouverture due à la capsulorhexis) peut adhérer sur le pourtour de l'optique provoquant la création d'une chambre fermée entre l'optique de l'implant et la partie postérieure du sac qui se remplit petit à petit de matière blanchâtre et non transparente. Ce phénomène demande également d'intervenir ultérieurement en découpant au moyen du laser YAG cette partie postérieure. Cette complication est appelée "after cataract" (AJO avril 1998 p. 426).

Il est connu par le EP-A-732 090 un implant en deux pièces, à savoir une pièce annulaire et une pièce centrale optique logée dans la pièce annulaire. La pièce centrale est semblable à tous les implants connus tandis que la pièce annulaire joue un rôle de tendeur du sac capsulaire.

Par la présente invention, on entend améliorer cet implant en deux parties de sorte que chacune d'elle est, une fois pliée, d'une taille suffisamment faible pour pouvoir être aisément introduite dans l'incision de la phaco-émulsification tout en conservant de grandes qualités de mise en place et de maintien de l'implant dans le sac capsulaire.

A cet effet, l'invention a donc pour objet un implant intraoculaire destiné à former un cristallin artificiel logé dans le sac capsulaire, qui comporte une première pièce en forme d'anneau réalisé en un matériau souple et pliable, dont la surface extérieure est sensiblement identique à la surface interne du sac capsulaire dans son état naturel (diamètre entre 9 et 10 mm) dans la région de son équateur et dont la surface intérieure définit une gorge, l'anneau présentant dans sa région de l'équateur une partie résistante à la compression radiale bordée de chaque côté par des parties en forme de lèvres tournées vers l'intérieur de l'anneau ; l'implant comporte également une seconde pièce, centrale, en matériau souple et pliable, en forme de disque biconvexe (partie optique) bordé en périphérie par une partie haptique dont les bords extrêmes sont à l'état libre inscrits dans un cercle de diamètre au plus égal au plus grand diamètre de la gorge. Ce diamètre est compris entre 7 et 9 mm. De manière préférée l'épaisseur des parties haptiques à leur extrémité est sensiblement égale à la largeur de la gorge mesurée au diamètre extérieur de la pièce centrale, pour empêcher tout mouvement antéro-postérieur de la seconde pièce par rapport à la première.

L'anneau peut être plié de manière à présenter une très faible dimension (deux ou quatre fois la plus grande dimension de sa section) ce qui permet une introduction aisée à travers une incision de petite dimension. En outre la pièce centrale de l'implant est de dimensions beaucoup plus réduites que celles des implants usuels et peut donc être également aisément introduite. Enfin, une fois logée à l'intérieur de l'anneau, dans sa gorge, la pièce centrale de l'implant ne subit plus aucune contrainte de la part du sac capsulaire si bien qu'aucune déformation ne peut l'affecter, qui en modifierait ses caractéristiques optiques. De ce fait, cette pièce centrale peut être réalisée dans un matériau plus souple que celui utilisé couramment et dans des épaisseurs plus faibles. En outre on peut, s'il le faut, changer la pièce centrale par une opération chirurgicale sans grand risque car on ne touche plus au sac capsulaire.

Dans un mode de réalisation, l'anneau comprend une armature interne en forme d'un ou deux joncs en matériau élastique qui coopère au meilleur dépliement de l'anneau dans le sac capsulaire et à une meilleure tenue de l'anneau à l'encontre des efforts nés de la contraction du sac capsulaire. Dans une variante de réalisation l'anneau peut comporter deux fentes équatoriales qui laissent la possibilité de mettre en place un implant à anses, lesquelles anses seraient logées dans lesdites fentes.

Enfin, dans une autre variante de réalisation, les lèvres de la partie annulaire sont dissymétriques, la lèvre postérieure étant massive alors que la lèvre antérieure qui se situe derrière l'iris est plus fine et peut venir se rabattre contre la lèvre postérieure massive en y pinçant la partie haptique de la pièce centrale, ce qui augmente sa stabilité.

D'autres caractéristiques et avantages ressortiront de la description donnée ci-après d'un mode de réalisation de l'invention.

Il sera fait référence aux dessins annexés parmi lesquels :
- la figure 1 est une vue en perspective générale d'un implant conforme à l'invention, la pièce centrale étant logée à l'intérieur de l'anneau périphérique de cet implant,
- la figure 2 est une vue en coupe selon le plan II-II de la figure 1,
- la figure 3 est une vue en plan d'une variante de réalisation, notamment de la partie centrale de l'implant,
- la figure 4 est une vue en coupe comparative d'un implant usuel avec la pièce centrale de l'implant de l'invention,
- la figure 5 illustre une variante de réalisation de la pièce annulaire,
- la figure 6 est une vue en coupe d'une variante de réalisation de l'implant selon l'invention.

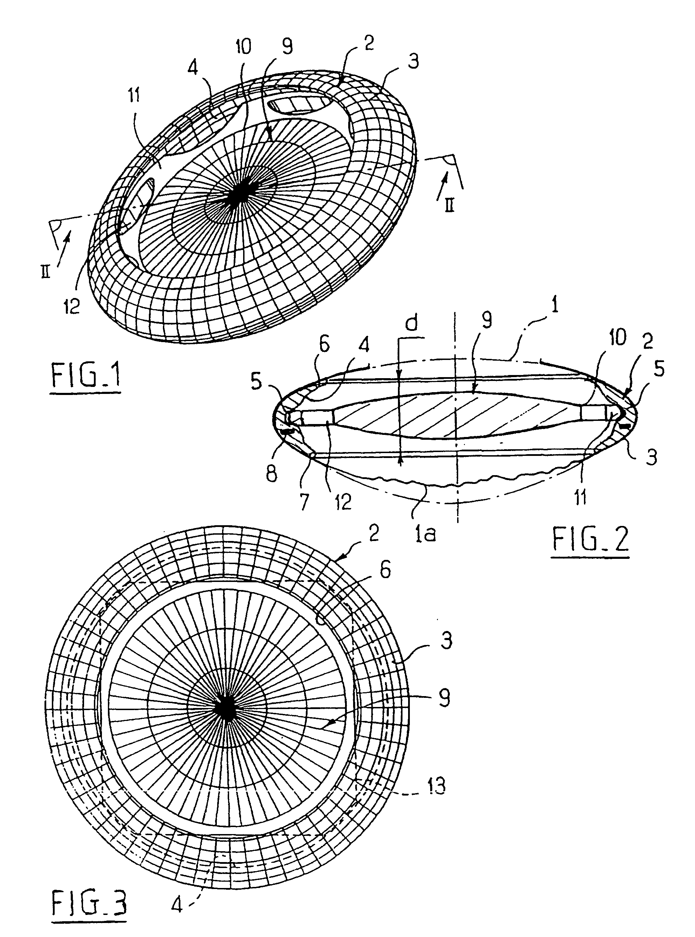

A la figure 2, on a représenté en trait mixte 1 la forme du sac capsulaire lorsqu'il constitue l'enveloppe du cristallin naturel d'un patient jeune (25-30 ans). L'implant selon l'invention représenté aux figures 1 et 2 comporte un anneau 2 dont la surface extérieure 3 est conformée de manière à être sensiblement identique à la forme du sac capsulaire 1 dans sa région équatoriale, c'est-à-dire dans sa région de plus grand diamètre. Son plus grand diamètre, dans cette région est égal(entre 9 et 10,5 mm) à celui du sac capsulaire non pas au moment de l'opération de la cataracte mais inférieur à cette valeur pour tenir compte du grossissement du cristallin naturel avec l'âge et donc rétablir le sac dans une dimension correspondant à celle d'un patient jeune.

La surface intérieure de cet anneau définit une gorge 4 ou un V dont le fond est ménagé dans une partie équatoriale 5 massive de l'anneau 2 avec deux lèvres 6 et 7 qui s'étendent de chaque côté de la partie 5. Cette partie 5 forme une partie circulaire apte à encaisser la rétraction du sac capsulaire pour le maintenir à un diamètre prédéterminé plus petit que celui qu'il avait adopté avant l'opération. Les lèvres 6 et 7 sont tournées vers l'intérieur de l'anneau et on notera que le bord interne de chaque lèvre délimite un cercle de 7 mm de diamètre environ et la distance d antéro-postérieure entre ces lèvres est de supérieure à l'épaisseur de la pièce centrale de l'implant. Cette distance sera comprise entre 1,1 et 2,25 mm.

Le matériau dans lequel est réalisé cet anneau est un matériau souple et pliable, par exemple une matière acrylique, un hydrogel ou du silicone qui possède une élasticité suffisante pour, d'une part recouvrer sa forme initiale lorsque l'anneau est introduit dans le sac capsulaire après avoir été plié et, d'autre part résister aux efforts de contraction qu'il subit de la part du sac capsulaire. Pour augmenter ces propriétés, on peut de manière avantageuse introduire à l'intérieur de l'anneau 2 une armature en forme d'un ou de deux joncs 8 qui peuvent affecter une section circulaire ou une section du genre rondelle plate comme représenté, les faces parallèles de cette rondelle étant parallèles au plan équatorial de l'anneau. Le matériau de ce jonc sera de préférence élastiquement déformable, du type PMMA ou en PROLENE (marque déposée) qui est un polyamide bien toléré par l'oeil.

L'implant selon l'invention comporte également une pièce centrale 9 qui porte la partie optique de celui-ci et qui de ce fait affecte la forme générale d'un disque biconvexe. Le matériau dans lequel est réalisée cette pièce centrale est également un matériau souple et déformable du genre hydrogel, acrylique ou silicone. Cette pièce centrale, outre le disque proprement dit dont la circonférence est notée 10 aux dessins, possède des pattes radiales ou des protubérances 11 qui viennent se loger à l'intérieur de la gorge 4 de l'anneau. L'extrémité de ces pattes 11 est circonscrite dans un cercle dont le diamètre est sensiblement égal ou légèrement inférieur au diamètre du fond de cette gorge. L'épaisseur e des pattes radiales 11 à leur extrémité est égale à la largeur de la gorge 4 à cet endroit, ce qui constitue une immobilisation antéro-postérieure de la pièce centrale.

Aux figures 1 et 2, les pattes 11 sont pourvues d'orifices 12 qui permettent notamment de faire tourner la pièce centrale de l'implant dans l'anneau si ce mouvement est nécessaire lors de sa mise en place.

A la figure 3, la pièce centrale 9 de l'implant possède également un disque central dont la périphérie est notée 10 et des pattes radiales 13 qui donnent à l'implant une forme carrée dont les coins sont abattus. Bien entendu toute autre forme de pièce centrale d'implant peut être accommodée à l'intérieur de l'anneau 2.

Dans d'autres modes de réalisation non représentés, la partie haptique de la pièce principale peut être formée par un voile en couronne dont la circonférence externe est logée dans la gorge. Dans ce cas, pour éviter la formation d'une chambre fermée derrière l'implant, ce voile sera percé d'ouvertures. Dans certains autres cas enfin, l'anneau peut présenter en fond de gorge des fentes ou des lumières traversantes 4a représentées à la figure 5 - par exemple deux lumières opposées sur 80°-90° d'angle - qui permettront d'accommoder une partie optique ayant des anses filaires pouvant se loger dans les fentes pour prendre directement appui sur le sac capsulaire.

Pour la mise en place de l'implant, le chirurgien procède, après avoir retiré le contenu du sac, à la mise en place de l'anneau 2 au travers de l'ouverture qui a servi à l'introduction des appareils de phaco-émulsification, cet anneau 2 étant replié en deux ou en quatre et introduit dans le sac capsulaire au moyen d'un outil tubulaire ou d'une pince appropriée. Le chirurgien contrôle son dépliement à l'intérieur de ce sac, et ajuste sa mise en place au niveau de l'équateur de celui-ci. Il procède ensuite à la mise en place de la pièce centrale 9 de l'implant, elle-même pliée ou roulée en forme tubulaire, et enfin lorsque cette pièce centrale est dépliée à l'intérieur de l'anneau 2, ajuste sa place de manière qu'elle se trouve dans la gorge 4 de l'anneau mis en place dans le sac capsulaire.

A titre indicatif, on notera que l'anneau possède un diamètre extérieur de l'ordre de 9 à 10,5 mm alors que la pièce centrale de l'implant a une dimension diagonale de l'ordre de 7 à 9 mm, ce qui est très inférieur aux implants connus. L'anneau 2, outre sa fonction de maintien du sac capsulaire dans un état le plus favorable puisque proche de son état naturel correspondant à un âge jeune (25 - 30 ans) du patient, constitue un protecteur pour la pièce centrale qui ne subit aucune contrainte radiale. Il est donc possible de donner aux parties haptiques de l'implant toute forme et toute dimension possible du moment que celles-ci assurent son centrage à l'intérieur de l'anneau.

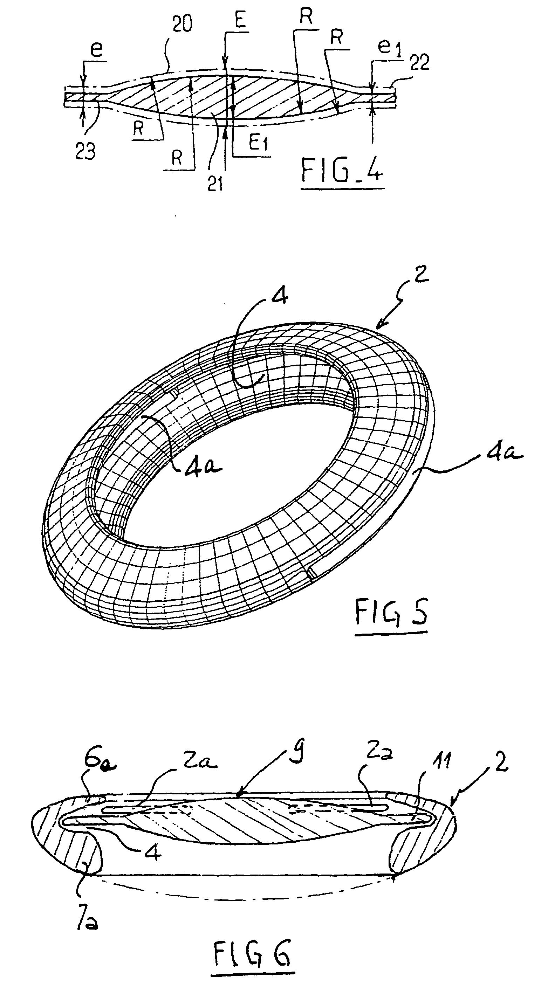

En particulier, la figure 4 est un schéma comparatif entre un implant usuel 20 et la pièce centrale d'implant 21 conforme à l'invention. Dans un implant usuel, la racine de la partie haptique 22 au voisinage du bord du disque optique doit avoir en général une épaisseur e de l'ordre de 0,4 mm. Le rayon de courbure R de chacune des faces du disque optique est de l'ordre de 12 mm, si bien que l'épaisseur E maximale de cette partie optique est de 1,15 mm.

Dans la partie centrale d'un implant conforme à l'invention avec les mêmes rayons de courbure, on peut se satisfaire d'une racine de partie haptique 23 d'épaisseur e1 de l'ordre de 0,2 mm, ce qui fait que l'épaisseur E1 de l'implant au centre de la partie haptique est de l'ordre de 0,95 mm. Grâce à l'invention, il est également possible de réaliser des implants dont la partie centrale peut avoir une partie optique de diamètre plus important que les implants usuels du fait de cette faible épaisseur des zones haptiques.

En se reportant enfin à la figure 6, on constate que la pièce annulaire 2 définit une gorge interne 4 entre deux lèvres 6a et 7a dissymétriques. La lèvre inférieure (postérieure dans l'oeil) 7a est massive et épouse la partie postérieure et équatoriale du sac capsulaire. La lèvre supérieure 6a (antérieure dans l'oeil derrière l'iris) est souple et, sous l'action de la contraction du sac capsulaire et de l'écrasement naturel de la partie équatoriale du sac, tend à être appliquée contre la lèvre 7a en refermant la gorge 4 et en assujettissant de manière stable les parties haptiques 11 de la pièce centrale 9. On notera que la lèvre supérieure 6a possède des ouvertures 2a pour d'une part aider la manipulation de l'anneau et d'autre part laisser libre une communication entre la chambre antérieure et la chambre postérieure de l'oeil.

## Revendications

1. Implant intraoculaire destiné à former un cristallin artificiel logé dans le sac capsulaire (1) de l'oeil, comportant une première pièce (2) en forme d'anneau, réalisée en un matériau souple et pliable, dont la surface intérieure est en forme de gorge (4) présentant ainsi dans la région de l'équateur une partie (5) résistante à la compression radiale bordée de chaque côté par des parties (6, 7) en forme de lèvres tournées vers l'intérieur de l'anneau, et une seconde pièce (9), en matériau souple et pliable, en forme de disque biconvexe bordé en périphérie par une partie haptique (11, 13), **caractérisé en ce que** la surface externe (3) de la première pièce (2) est sensiblement identique à la surface interne du sac capsulaire (1) à l'état naturel d'un patient jeune dans la région de son équateur, et **en ce que** les bords extrêmes de ladite partie haptique sont à l'état libre inscrits dans un cercle de diamètre au plus égal au diamètre en fond de gorge (4).

2. Implant selon la revendication 1, **caractérisé en ce que** la distance (d) séparant les lèvres (6, 7) mesurée dans le sens antéro-postérieur est supérieure à l'épaisseur maximale de la seconde pièce (9) de l'implant.

3. Implant selon la revendication 2, **caractérisé en ce que** cette distance est comprise entre 1,1 mm et 2,25 mm.

4. Implant selon l'une des revendications précédentes, **caractérisé en ce que** la partie haptique est formée par des pattes ou protubérances radiales (11, 13) réparties autour de la périphérie de la partie optique de la seconde pièce.

5. Implant selon l'une des revendications précédentes, **caractérisé en ce que** le diamètre extérieur de l'anneau (2) est compris entre 9 et 10,5 mm tandis que son diamètre intérieur le plus grand est compris entre 7 et 9 mm.

6. Implant selon l'une des revendications précédentes, **caractérisé en ce que** l'anneau (2) comporte une armature (8) noyée en forme de jonc en matériau élastique.

7. Implant selon la revendication 6, **caractérisé en ce que** le jonc (8) est une rondelle plane à faces parallèles entre elles et au plan équatorial de l'anneau (2).

8. Implant selon l'une quelconque des revendications précédentes, **caractérisé en ce que** l'anneau (2) comporte en fond de gorge des fentes ou lumières traversantes (4a).

9. Implant selon l'une quelconque des revendications précédentes, **caractérisé en ce que** la première pièce annulaire présente deux lèvres dissymétriques, l'une (6a) antérieure fine susceptible d'être rabattue sur l'autre (7a) postérieure massive en refermant entre elles la gorge (4) sur la partie haptique (11) de la pièce centrale (9).

## Claims

1. An intraocular implant for forming an artificial lens received in the capsular bag (1) of the eye, comprising a first part (2) in the form of an annulus, made of a material that is flexible and foldable whose inside surface is in the form of a groove (4) such that the annulus presents in its equator region a portion (5) that withstands radial compression and that is disposed between portions (6, 7) in the form of lips directed towards the inside of the annulus, and a second part (9) of a material that is flexible and foldable, in the form of a biconvex disk having a haptic portion (11, 13), **characterized in that** the outside surface (3) of said first part (2) in the region of its equator is substantially identical to the inside surface of the capsular bag (1) in the natural state for a young patient, and **in that** the outermost edges of said haptic portion are in the free state inscribed in a circle of diameter no greater than the diameter of the bottom of the groove (4).

2. An implant according to claim 1, **characterized in that** the distance (d) between the lips (6, 7) measured in the antero-posterior direction is greater than the maximum thickness of the second part (9) of the implant.

3. An implant according to claim 2, **characterized in that** said distance lies in the range 1.1 mm to 2.25 mm.

4. An implant according to claims 1 to 3, **characterized in that** the haptic portion is formed by radial projections or tabs (11, 13) distributed around the periphery of the optical portion of the second part.

5. An implant according to claim 1, **characterized in that** the outside diameter of the annulus (2) lies in the range 9 mm to 10.5 mm while its largest inside diameter lies in the range 7 mm to 9 mm.

6. An implant according to claim 1, **characterized in that** the annulus (2) includes embedded reinforcement (8) in the form of at least one ring of resilient material.

7. An implant according to claim 6, **characterized in that** the ring (6) is a plane washer having faces that are parallel to each other and to the equatorial plane of the annulus (2).

8. An implant according to claim 1, **characterized in that** the annulus (2) has through slots or openings (4a) in the bottom of its groove.

9. An implant according to claim 1, **characterized in that** the first annular part has two asymmetrical lips, comprising a fine anterior lip (6a) suitable for being folded back against the posterior other lip (7a) which is thick, the lips between them closing the groove (4) onto the haptic portion (11) of the central part (9).

## Patentansprüche

1. Intraokulares Implantat zum Bilden einer künstlichen Linse, die im Kapselsack (1) des Auges sitzt, mit einem ersten Teil (2) in Form eines aus einem weichen und biegsamen Material hergestellten Ringes, dessen innere Oberfläche in Form einer Hohlkehle (4) ausgebildet ist, derart, daß sie in der Region des Äquators eine gegenüber einer radialen Komprimierung widerstandsfähige Partie (5) aufweist, die an jeder Seite durch Partien (6, 7) in Form von dem Inneren des Ringes zugewandten Lippen berandet ist, und mit einem zweiten Teil (9) aus einem weichen und biegsamen Material in Form einer bikonvexen Scheibe, die am Umfang von einer haptischen Partie (11, 13) umrandet ist, **dadurch gekennzeichnet, daß** die äußere Oberfläche (3) des ersten Teils (2) in der Region seines Äquators im Wesentlichen identisch ist mit der inneren Oberfläche des Kapselsackes (1) im natürlichen Zustand eines jungen Patienten, und daß die äußersten Ränder der haptischen Partie im freien Zustand einen Kreis mit einem Durchmesser höchstens gleich dem Durchmesser am Boden der Hohlkehle (4) umschreiben.

2. Implantat nach Anspruch 1, **dadurch gekennzeichnet, daß** der die Lippen (6, 7) trennende Abstand (d), gemessen in der Richtung anterior - posterior, größer als die maximale Dicke des zweiten Teils (9) des Implantats ist.

3. Implantat nach Anspruch 2, **dadurch gekennzeichnet, daß** dieser Abstand zwischen 1,1 mm und 2,25 mm beträgt.

4. Implantat nach einem der vorstehenden Ansprüche, **dadurch gekennzeichnet, daß** die haptische Partie durch radiale Patten oder Protuberanzen (11, 13) gebildet wird, die um den Umfang der optischen Partie des zweiten Teils verteilt sind.

5. Implantat nach einem der vorstehenden Ansprüche, **dadurch gekennzeichnet, daß** der Außendurchmesser des Ringes (2) zwischen 9 und 10,5 mm beträgt, während sein größter Innendurchmesser zwischen 7 und 9 mm beträgt.

6. Implantat nach einem der vorstehenden Ansprüche, **dadurch gekennzeichnet, daß** der Ring (2) eine Armierung (8) aufweist, die in Form eines Reifes aus elastischem Material eingebettet ist.

7. Implantat nach Anspruch 6, **dadurch gekennzeichnet, daß** der Reif (8) eine ebene Scheibe mit zueinander und zu der Äquatorebene des Ringes (2) parallelen Flächen ist.

8. Implantat nach einem der vorstehenden Ansprüche, **dadurch gekennzeichnet, daß** der Ring (2) am Boden der Hohlkehle quer verlaufende Schlitze oder Öffnungen (4a) aufweist.

9. Implantat nach einem der vorstehenden Ansprüche, **dadurch gekennzeichnet, daß** das erste ringförmige Teil zwei asymmetrische Lippen aufweist, eine (6a) feine anterior liegende, die dazu geeignet ist, auf die andere (7a) massive posterior liegende umgeschlagen zu werden, derart, daß zwischen diesen die Hohlkehle (4) über der haptischen Partie (11) des zentralen Teils (9) geschlossen wird.
